(19) Europäisches Patentamt / European Patent Office / Office européen des brevets

(11) **EP 3 139 166 A1**

(12) **EUROPEAN PATENT APPLICATION**
published in accordance with Art. 153(4) EPC

(43) Date of publication:
**08.03.2017 Bulletin 2017/10**

(51) Int Cl.:
*G01N 33/566* [(2006.01)]  *C12M 1/34* [(2006.01)]
*G01N 33/543* [(2006.01)]  *G01N 33/574* [(2006.01)]
*C12Q 1/04* [(2006.01)]

(21) Application number: **15786712.8**

(22) Date of filing: **30.04.2015**

(86) International application number:
**PCT/JP2015/062955**

(87) International publication number:
**WO 2015/166977 (05.11.2015 Gazette 2015/44)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **02.05.2014 JP 2014095099**

(71) Applicants:
• **Osaka Prefecture University Public Corporation
Osaka 599-8531 (JP)**

• **Sharp Kabushiki Kaisha
Sakai City, Osaka 590-8522 (JP)**

(72) Inventors:
• **TOKONAMI, Shiho
Sakai-shi
Osaka 599-8531 (JP)**
• **NAKASE, Ikuhiko
Sakai-shi
Osaka 599-8531 (JP)**

(74) Representative: **Müller Hoffmann & Partner
Patentanwälte mbB
St.-Martin-Strasse 58
81541 München (DE)**

(54) **POLYMER FILM FOR DETECTION OF CANCER CELLS AND MANUFACTURING METHOD THEREOF, AND CANCER CELL DETECTION DEVICE USING SUCH POLYMER FILM**

(57)    A polymer membrane for cancer cell detection having a surface provided with a mold having a three-dimensional structure complementary to a portion of a steric structure of a cancer cell to be detected; a method of producing the same; and a cancer cell detection device including the polymer membrane are provided. The polymer membrane for cancer cell detection can be obtained, for example, by a producing method including: polymerizing monomers in presence of cancer cells to be detected, to form a cancer cell-containing polymer membrane having the cancer cells incorporated therein; and removing at least part of the cancer cells incorporated in the cancer cell-containing polymer membrane.

FIG.3

EP 3 139 166 A1

**Description**

TECHNICAL FIELD

The present invention relates to a polymer membrane allowing specific detection of a cancer cell as a target to be detected, and a method of producing the polymer membrane. The present invention also relates to a cancer cell detection device including the polymer membrane.

BACKGROUND ART

It is currently said that the leading cause of death of Japanese people is malignant neoplasms such as cancers. Thus, it is significantly important to detect a cancer cell at an early stage for the purpose of decreasing the mortality rate. "Cancers" generally mean malignant tumors. Depending on the originating tissues or the types of cells, such "cancers" are classified into "carcinoma" originating from epithelial cells, "sarcoma" derived from connective tissues or muscle cells, "leukemia" originating from hematopoietic cells, those originating from neural cells, and the like.

Cancer diagnosis has been conventionally performed by: (1) a positron emission tomography (PET) method of detecting cancer cells from the outside by images of radioactive rays, which are taken by a special camera, emitted from a radiation-emitting test drug injected in advance and concentrated in a portion where a cancer exists; (2) a computed tomography (CT) method of detecting cancer cells by imaging the inside of the body from photographs of the cross-section of the body taken using an X-ray; (3) a circulating tumor cell (CTC) test method of detecting circulating tumor cells (CTC) in blood using a labeled substance like a fluorescence staining agent or an antigenic marker; and the like.

However, it has been pointed out that the PET method and the CT method pose a lot of problems, for example, that there may be a risk of radiation exposure; much time and costs may be required for conducting tests; a large-scale device may be required; a special engineer may be required; and the like. In contrast, the CTC test method is advantageous that there is no risk of radiation exposure and a large-scale device is not required, but this CTC test method employs labeled substances, which essentially requires skilled technicians for conducting tests. Thus, the CTC test method still needs to be improved for the sake of convenience of conducting tests.

WO2012/121229 (PTD 1) discloses that, as a sensor not for detecting cells like cancer cells but for detecting microorganisms represented by bacteria, a polymer layer is used that includes a mold having a three-dimensional structure complementary to the steric structure of a microorganism to be detected.

CITATION LIST

PATENT DOCUMENT

PTD 1: WO2012/121229

SUMMARY OF INVENTION

TECHNICAL PROBLEM

In view of the above-described conventional problems, an object of the present invention is to provide a polymer membrane for cancer cell detection that allows immediate and easy detection of cancer cells in an unlabeled manner, and a method of producing the polymer membrane. Another object of the present invention is to provide a cancer cell detection device including the polymer membrane.

SOLUTION TO PROBLEM

The above-described PTD 1 discloses that, when a microorganism represented by bacteria is designated as a target to be detected, a polymer membrane having a mold of the microorganism is excellent in detection specificity. However, when a target to be detected is a cancer cell that is significantly larger in size (approximately an order of magnitude larger) and softer as a substance than bacteria, a person skilled in the art cannot foresee: (i) whether or not a polymer membrane having a mold of a cancer cell can be produced as in the case of bacteria; and (ii) whether or not this polymer membrane shows excellent detection specificity for a cancer cell (characteristics of specifically detecting a cancer cell to be detected). Rather, in light of the above-described difference between bacteria and a cancer cell, the persons skilled in the art generally predict that it may be difficult to implement a polymer membrane having a mold of a cancer cell and to develop excellent detection specificity for a cancer cell.

The present invention is based on revolutionary study results: that it becomes possible to actually implement a polymer membrane provided with a mold having a three-dimensional structure complementary to a portion of the steric structure of a cancer cell to be detected; and that this polymer membrane exhibits excellent detection specificity, each of these study results overcoming the above-described ordinary prediction by the persons skilled in the art. In other words, the present invention provides a polymer membrane for cancer cell detection, a cancer cell detection device, and a method of producing the polymer membrane for cancer cell detection, as will be described below.

[1] A polymer membrane for cancer cell detection is provided. The polymer membrane has a surface provided with a mold having a three-dimensional structure complementary to a portion of a steric structure of a cancer cell to be detected.

[2] In the polymer membrane according to [1], a thickness of the polymer membrane is 5 μm or less.

[3] The polymer membrane according to [1] or [2] is obtained by a producing method including:

    polymerizing monomers in presence of cancer cells to be detected, to form a cancer cell-containing polymer membrane having the cancer cells incorporated therein; and

    removing at least part of the cancer cells incorporated in the cancer cell-containing polymer membrane.

[4] In the polymer membrane according to [3], the removing includes, in a following order:

    decreasing a volume of the cancer cells incorporated in the cancer cell-containing polymer membrane or at least partially neutralizing an electrostatic interaction between the cancer cells and a polymer membrane forming the cancer cell-containing polymer membrane; and peroxidizing the cancer cell-containing polymer membrane to release at least part of the cancer cells incorporated in the cancer cell-containing polymer membrane.

[5] In the polymer membrane according to [4], the decreasing a volume of the cancer cells incorporated in the cancer cell-containing polymer membrane or at least partially neutralizing an electrostatic interaction between the cancer cells and a polymer membrane forming the cancer cell-containing polymer membrane includes treating the cancer cell-containing polymer membrane with heparin sodium.

[6] In the polymer membrane according to any one of [3] to [5], the monomers are selected from the group consisting of pyrrole, aniline, thiophene, and a derivative thereof.

[7] A cancer cell detection device includes: a detection unit including the polymer membrane according to any one of [1] to [6]; a guiding unit for guiding a cancer cell to be detected, which is contained in a sample solution, toward the surface of the polymer membrane; and a sensing unit for sensing a state where the cancer cell to be detected is captured in the mold.

[8] In the cancer cell detection device according to [7], the guiding unit is configured to apply an alternating-current (AC) voltage between a pair of electrodes to guide the cancer cell to be detected toward the surface of the polymer membrane by dielectrophoresis.

[9] In the cancer cell detection device according to [7] or [8], the sensing unit includes a crystal oscillator. The sensing unit is configured to measure a mass change of the polymer membrane based on a change of a resonance frequency of the crystal oscillator, to sense the state where the cancer cell to be detected is captured.

[10] A method of producing a polymer membrane for cancer cell detection is provided. The polymer membrane for cancer cell detection has a surface provided with a mold having a three-dimensional structure complementary to a portion of a steric structure of a cancer cell to be detected. The method includes: polymerizing monomers in presence of cancer cells to be detected, to form a cancer cell-containing polymer membrane having the cancer cells incorporated therein; and removing at least part of the cancer cells incorporated in the cancer cell-containing polymer membrane.

[11] In the producing method according to [10], the removing includes, in a following order:

    decreasing a volume of the cancer cells incorporated in the cancer cell-containing polymer membrane or at least partially neutralizing an electrostatic interaction between the cancer cells and a polymer membrane forming the cancer cell-containing polymer membrane; and peroxidizing the cancer cell-containing polymer membrane to release at least part of the cancer cells incorporated in the cancer cell-containing polymer membrane.

[12] In the producing method according to [11], the decreasing a volume of the cancer cells incorporated in the cancer cell-containing polymer membrane or at least partially neutralizing an electrostatic interaction between the cancer cells and a polymer membrane forming the cancer cell-containing polymer membrane includes treating the cancer cell-containing polymer membrane with heparin sodium.

ADVANTAGEOUS EFFECTS OF INVENTION

According to the present invention, it becomes possible to provide a polymer membrane for cancer cell detection that allows immediate and easy detection of a specific cancer cell in an unlabeled manner (that is, directly without using a labeled substance), and a cell detection device including the polymer membrane. Also, according to the present invention, it becomes possible to provide a polymer membrane for cancer cell detection that allows specific detection of a cancer cell to be detected (with excellent selectivity), and a cell detection device including the polymer membrane.

BRIEF DESCRIPTION OF DRAWINGS

Fig. 1 is a cross-sectional view schematically showing a polymer membrane for cancer cell detection according to the present invention.

Fig. 2 is a cross-sectional view schematically showing the state where cancer cells are captured in molds of the polymer membrane for cancer cell detection shown in Fig. 1.

Fig. 3 is a cross-sectional view schematically showing an example of a method of producing a polymer membrane for cancer cell detection according to the present invention, including cross-sectional views of: (a) before a polymerization step; (b) after the polymerization step; and (c) after a cancer cell removal step.

Fig. 4 is a schematic diagram showing the schematic configuration of a suitable example of a cancer cell detection device according to the present invention.

Fig. 5 is an electron micrograph of leukemia cells CCRF-CEM.

Fig. 6 is an electron micrograph of the surface of a polypyrrole film (a cancer cell-containing polymer membrane) after the polymerization step in Example 1.

Fig. 7 is a graph showing the relation between the resonance frequency of a crystal oscillator and the time in the polymerization step in Example 1.

Fig. 8 is an electron micrograph of the surface of a peroxidized polypyrrole film (a polymer membrane for cancer cell detection) obtained after a peroxidation step in Example 1.

Fig. 9 is a graph showing the relation between the resonance frequency of the crystal oscillator and the time in the peroxidation step in Example 1.

Fig. 10 is a graph showing results of a cancer cell detection experiment performed using the cancer cell detection device in Example 1, and also showing the relation between the AC-voltage application time and the resonance frequency of the crystal oscillator during detection of cancer cells.

Fig. 11 is a graph showing results of the cancer cell detection experiment performed using the cancer cell detection device in Example 1, and also showing a comparison between: a resonance frequency change ∆F for 300 seconds of the AC-voltage application time in the case where a cancer cell is a target cancer cell CCRF-CEM; and a resonance frequency change ∆F for 300 seconds of the AC-voltage application time in the case where a cancer cell is a non-target cancer cell THP-1.

Fig. 12 is an electron micrograph of a leukemia cell THP-1.

Fig. 13 is an electron micrograph of the surface of a polypyrrole film (a cancer cell-containing polymer membrane) obtained after the polymerization step in Example 2.

Fig. 14 is a graph showing the relation between the resonance frequency of a crystal oscillator and the time in a polymerization step in Example 2.

Fig. 15 is an electron micrograph of the surface of a peroxidized polypyrrole film (a polymer membrane for cancer cell detection) obtained after the peroxidation step in Example 2.

Fig. 16 is a graph showing the relation between the resonance frequency of the crystal oscillator and the time in the peroxidation step in Example 2.

Fig. 17 is a graph showing results of a cancer cell detection experiment performed using a cancer cell detection device in Example 2, and also showing the relation between the AC-voltage application time and the resonance frequency of the crystal oscillator during detection of cancer cells.

Fig. 18 is a graph showing results of the cancer cell detection experiment performed using the cancer cell detection device in Example 2, and also showing a comparison between; a resonance frequency change ∆F for 300 seconds of the AC-voltage application time in the case where a cancer cell is a target cancer cell THP-1; and a resonance frequency change ∆F for 300 seconds of the AC-voltage application time in the case where a cancer cell is a non-target cancer cell CCRF-CEM.

DESCRIPTION OF EMBODIMENTS

<Polymer Membrane for Cancer Cell Detection and Method of Producing the Same>

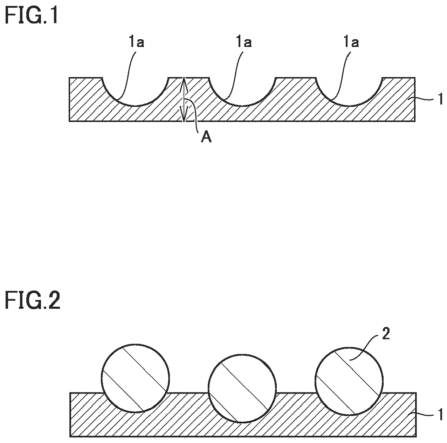

Fig. 1 is a cross-sectional view schematically showing a polymer membrane for cancer cell detection according to the present invention. As shown in Fig. 1, a polymer membrane for cancer cell detection 1 according to the present invention has a surface provided with a plurality of molds 1a each having a three-dimensional structure complementary to a portion of a steric structure of a cancer cell to be detected (which will be hereinafter also referred to as a "target cancer cell"). Polymer membrane for cancer cell detection 1 incorporates a target cancer cell in its mold 1a and captures this cancer cell, thereby detecting this cancer cell. Fig. 2 shows a schematic cross-sectional view of the state where target cancer cells 2 are captured in molds 1a of polymer membrane for cancer cell detection 1.

In this way, polymer membrane for cancer cell detection 1 according to the present invention is provided with mold 1a having a three-dimensional structure complementary to a portion of the steric structure of target cancer cell 2. Accordingly, this polymer membrane for cancer cell detection 1 captures target cancer cell 2 with excellent selectivity, but captures none or little of cancer cells and other substances that each are not complementary to the three-dimensional structure of this mold 1a. Thus, this polymer membrane for cancer cell detection 1 is excellent in capture selectivity. Due to such characteristics, target cancer cells 2 can be specifically detected.

Though not clear at this stage, the term "complementary" used herein may include a meaning of being complementary with respect to the surface shape of target cancer cell 2 and/or being complementary with respect to the molecular level structure of the surface of target cancer cell 2.

Polymer membrane for cancer cell detection 1 can have a thickness, for examples, of about 0.1 $\mu$m to 10 $\mu$m. Even the thickness of 5 $\mu$m or less or the thickness of 4 $\mu$m or less still allows excellent specificity for cancer cell detection. Since the size (the diameter or the greatest diameter) of the cancer cell is generally about 10 $\mu$m, mold 1a may have a three-dimensional structure complementary to a portion corresponding to about 50% or less of the steric structure of target cancer cell 2, or further may have a three-dimensional structure complementary to a portion corresponding to about 40% or less of the steric structure of target cancer cell 2. Furthermore, the thickness of 5 $\mu$m or less is also advantageous in terms of ease of production.

In addition, from the viewpoint of ease of production, handling ability, mechanical strength, and detection specificity for the polymer membrane for cancer cell detection 1, it is preferable that the thickness of polymer membrane for cancer cell detection 1 is 1 $\mu$m or more, and also preferable that mold 1a has a three-dimensional structure complementary to a part of the steric structure including a surface molecular structure of target cancer cell 2.

The thickness of polymer membrane for cancer cell detection 1 corresponds to a distance from a portion with no mold 1a in the surface provided with molds 1a to the surface that faces this portion of the surface (Fig. 1 shows a thickness of polymer membrane for cancer cell detection 1 by a reference character A). Thickness A can be measured by a scanning election microscope, a laser microscope, or a level difference measuring machine.

From the viewpoint of ease of production, mechanical strength, and independency of each mold 1a (for suppressing formation of another mold having a different shape by coupling of two or more molds 1a together), the number of molds 1a provided in polymer membrane for cancer cell detection 1 is preferably equal to or less than $9 \times 10^6$ per square centimeter of the surface having mold 1a formed thereon, more preferably equal to or less than $5 \times 10^6$, and further more preferably equal to or less than $3 \times 10^6$. Also, in order to achieve sufficient detection sensitivity for target cancer cells 2, the number of molds 1a is preferably equal to or more than $1 \times 10^3$ per square centimeter of the above-mentioned surface, and more preferably equal to or more than $1 \times 10^4$.

The number of molds 1a per square centimeter of the surface provided with molds 1a can be calculated from the number of molds 1a included in an SEM image of an area of 400 $\mu$m $\times$ 300 $\mu$m obtained by imaging an arbitrary portion of the surface.

The polymers forming a polymer membrane for cancer cell detection 1 are not particularly limited, but can be various types of polymers irrespective of whether conductive or non-conductive. However, when polymer membrane for cancer cell detection 1 is produced by the after-mentioned method that can be suitably employed, it is preferable to use, as a raw material, monomers forming a conductive polymer by a polymerization reaction in order to allow efficient formation of a polymer membrane having target cancer cells 2 incorporated therein as a production intermediate. Examples of monomers forming a conductive polymer may be pyrrole, aniline, thiophene, and derivatives thereof.

Examples of target cancer cells 2 may be cells originating from cancers described below: chronic leukemia, acute leukemia, malignant lymphoma, multiple myeloma, acute promyelocytic leukemia, acute myelogenous leukemia, chronic myelogenous leukemia, protopathic bone-marrow fibrosis; glioma, astrocytoma, brain stem glioma, ependymoma, oligodendroglioma, non-glioma, vestibular schwannoma, craniopharyngioma, medulloblastoma, meningioma, pinealoma, pineoblastoma, protopathic brain lymphoma brain tumor; pancreatic cancers like insulinoma, gastrinoma, glucagonoma, vipoma, somatostatin secreting tumor, and carcinoid or islet-of-Langerhans cell tumor; pituitary cancers like Cushing's disease, prolactin secreting tumor, acromegaly, and diabetes insipidus; eye cancers like eye melanoma (for example, iris melanoma, choroidal melanoma), and retinoblastoma; vaginal cancers like carcinoma of a squamous cell, glandular cancer and melanoma; thoracic cancers like glandular cancer, lobulus (small cell) cancer, intraductal cancer, mammary gland medullary cancer, thoracic mucous cancer, thoracic tubular cancer, thoracic papillary cancer, Paget's disease, and inflammatory breast cancer; sarcoma in osseous tissue and connective-tissue like bone sarcoma, osteosarcoma, chondrosarcoma, Ewing's sarcoma, malignant giant cell tumor, fibrosarcoma in bone, chordoma, periosteal osteosarcoma, parenchyma sarcoma, hemangiosarcoma, fibrosarcoma, Kaposi sarcoma, leiomyosarcoma, liposarcoma, lymphangiosarcoma, schwannoma, rhabdomyosarcoma, synovial sarcoma; vulva cancer like carcinoma in squamous cells, melanoma, glandular cancer, carcinoma in basal cell, sarcoma, and Paget's disease; uterine cervical cancers like carcinoma in squamous cells, and glandular cancer; uterine cancers like an endometrium cancer and uterus sarcoma; stomach cancers like glandular cancer, fungus-like (polyp-like), ulcer-like, superficial spreading type, sporadic spreading type, malignant lymphoma, liposarcoma, fibrosarcoma, and carcinosarcoma; colon cancer; rectal cancer; liver cancers like liver cell carcinoma and hepatoblastoma, and gall bladder cancer; papillary-like and nodular-like bile duct carcinoma; lung cancers like non-small cell lung cancer, squamous cell carcinoma (epidermoid carcinoma), glandular cancer, large cell carcinoma, and small cell lung cancer; adrenal cancers like pheochromocytoma and adrenal-cortex carcinoma; thyroid cancers like papillary- or follicular-thyroid cancer, medullary thyroid cancer and anaplastic thyroid cancer; ovarian cancers like ovarian epithelial carcinoma, a boundary region tumor, a germ cell tumor, and an interstitial tumor; testicle cancers like embryo tumor, seminoma, anaplastic, spermatocyte, non-seminoma, embryonal cancer, teratocarcinoma, choriocarcinoma (yolk sac tumor), prostatic cancer, leiomyosarcoma, and rhabdomyosarcoma; penile cancer; oral cancers like squamous cell carcinoma; renal cancers like renal cell cancer, glandular cancer, hypernephroma, fibrosarcoma, and transitional cell cancer; Wilms tumor; bladder cancers like transitional cell carcinoma, squamous cell carcinoma, glandular cancer, carcinosarcome; salivary gland cancers like glandular cancer, mucoepidermoid carcinoma, adenoid cystic carcinoma; pharyngeal cancers like squamous cell cancer and a wart; skin cancers like a basal cell carcinoma, squamous cell carcinoma and melanoma, superficial spreading melanoma, nodular melanoma, lentigo maligna melanoma, and distal-part lentigo melanoma; esophagus cancers like a squamous cancer, a glandular cancer, an adenoid cystic cancer, a mucoepidermoid carcinoma, an adenosquamous carcinoma, sarcoma, melanoma, plasmacytoma, wart carcinoma, and oat cell (small cell) carcinoma.

A preferable example of target cancer cell 2 is a cancer cell (tumor cell) circulating in blood. There may be two types or more of target cancer cells 2, in which case molds 1a corresponding to two or more types of target cancer cells 2 can be formed in polymer membrane for cancer cell detection 1. In addition, a cell containing a cancer cell has an outer surface including a carboxyl group and a sulfate group originating from sugar chains such as sialic acid, heparan sulfate, and chondroitin sulfate. Also, in liquid, the surface of the cell is applied with a negative electric charge by ionization of the carboxyl group and the sulfate group.

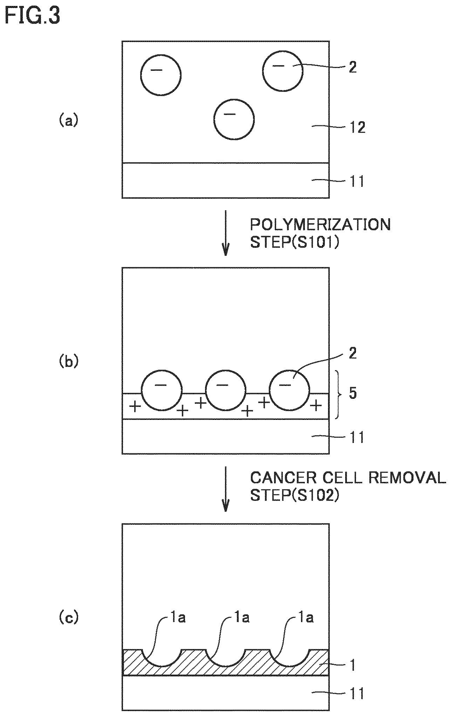

Then, referring to Fig. 3, the method of producing a polymer membrane for cancer cell detection 1 will be hereinafter described. Polymer membrane for cancer cell detection 1 can be suitably produced by a method including the following steps of: a polymerization step S101 of polymerizing monomers in the presence of target cancer cells 2 to form a cancer cell-containing polymer membrane 5 having target cancer cells 2 incorporated therein; and a cancer cell removal step S102 of removing at least part of target cancer cells 2 incorporated in cancer cell-containing polymer membrane 5.

In this producing method, first, as shown in Fig. 3(a), a first electrode 11 and a liquid 12 containing target cancer cells 2 and monomers (pyrrole and the like) are prepared. Then, liquid 12 is arranged on first electrode 11 so as to be in contact therewith. The monomer concentration in liquid 12 can be approximately 1 mM to 10 M while the number of target cancer cells 2 can be approximately 1 to $10^8$.

Then, in polymerization step S101, electrolysis is performed in the state where first electrode 11 is set as an anode while a counter electrode (not shown) is set as a cathode. Then, by the oxidative polymerization reaction (electropolymerization reaction) of monomers, cancer cell-containing polymer membrane 5 having target cancer cells 2 incorporated therein is formed on first electrode 11 (see Fig. 3(b)). For example, when a monomer is pyrrole, the polymer membrane formed on first electrode 11 is polypyrrole. In this polymerization step S101, monomers emit electrons to first electrode 11. Thus, in order to compensate for positive charges contained in the formed polymer, target cancer cells 2 each having a surface applied with a negative electric charge are efficiently incorporated in the polymer membrane as dopant anions.

By the polymerization time in polymerization step S101, adjustment of the monomer concentration in liquid 12 and the like, the thickness of the finally obtained polymer membrane for cancer cell detection 1 can be controlled. The number of molds 1a provided in polymer membrane for cancer cell detection 1 can be controlled by the concentration of target cancer cells 2 in liquid 12, the concentration ratio between the monomers and target cancer cells 2, and the like. Furthermore, the number of molds 1a can be controlled also by adjusting the removal rate of target cancer cells 2 (which will be hereinafter referred to as an "de-doping rate") in cancer cell removal step S102 described later.

Then, in cancer cell removal step S102, as shown in Fig. 3(c), at least part of target cancer cells 2 incorporated in cancer cell-containing polymer membrane 5 are removed, to obtain polymer membrane for cancer cell detection 1 provided with mold 1a having a three-dimensional structure complementary to a portion of the steric structure of target cancer cell 2. Polymer membrane for cancer cell detection 1 may be provided with partial molds 1a having target cancer cells 2 still incorporated therein without being removed therefrom.

Examples of the method of removing target cancer cells 2 may include:

   a) a method of using a medical agent to at least partially dissolve or destroy target cancer cells 2 incorporated in cancer cell-containing polymer membrane 5, to decrease the volume of target cancer cells 2, thereby removing target cancer cells 2;
   b) a method of utilizing osmotic pressure to decrease the volume of target cancer cells 2 incorporated in cancer cell-containing polymer membrane 5, thereby removing target cancer cells 2;
   c) a method of using a medical agent to at least partially neutralize the electrostatic interaction between target cancer cells 2 and the polymer membrane forming cancer cell-containing polymer membrane 5, thereby removing target cancer cells 2; and
   d) a method of performing one or more steps in the methods a) to c) and then peroxidizing cancer cell-containing polymer membrane 5, thereby removing target cancer cells 2.

According to the above-described methods a) and b), each of the incorporated target cancer cells 2 is reduced in size to facilitate emission of target cancer cells 2 from molds 1a. Examples of the medical agents used in the method a) may be a surfactant (non-ionic surfactant and the like), a chelate agent (EDTA, EGTA, NTA, DTPA, HEDTA and the like), and the like.

An example of the method b) may be a method of bringing a salt solution like sodium chloride into contact with cancer cell-containing polymer membrane 5. Examples of the medical agents used in the method c) may be: a cationic molecule that at least partially neutralizes the negative electric charges included in target cancer cell 2 incorporated in cancer cell-containing polymer membrane 5 (for example, polylysine, poly-arginine); and an anionic molecule that at least partially neutralizes the positive electric charges in the polymer membrane forming cancer cell-containing polymer membrane 5 (for example, heparin sodium).

By the peroxidation treatment in the method d), the polymer membrane of cancer cell-containing polymer membrane 5 becomes electrically neutral. Accordingly, target cancer cells 2 can be efficiently emitted (un-doped) from molds 1a. In the case where the polymer membrane of cancer cell-containing polymer membrane 5 is for example polypyrrole, the polymer membrane turns into peroxidated polypyrrole by this peroxidation treatment. The peroxidation treatment is useful when target cancer cells 2 cannot be effectively removed in any one or more treatments in the methods a) to c). However, this peroxidation treatment is not necessarily required when target cancer cells 2 can be effectively removed by any one or more treatments in the methods a) to c). In contrast, target cancer cells 2 may be removed only by the peroxidation treatment without having to perform the methods a) to c), if possible.

Since the peroxidation treatment causes curing of polymer membrane for cancer cell detection 1, this peroxidation treatment is advantageous also for fixing the three-dimensional structure of mold 1a formed by removal of each of target cancer cells 2. The peroxidation treatment can be performed by applying a voltage between first electrode 11 and the counter electrode (not shown), for example, in the state where the liquid in a range of neutral to alkaline is brought into contact with the surface of the polymer membrane (this liquid may be obtained by adjusting liquid 12 after polymerization step S101 so as to fall within a range from neutral to alkaline).

In addition, a heating treatment, an ultrasonic treatment or the like may be combined together with the treatments in the above-described methods a) to d) in cancer cell removal step S102.

First electrode 11 used for the electropolymerization reaction in polymerization step S101 is not particularly limited, but for example may be a gold electrode, a multilayer electrode formed of gold and chromium, a multilayer electrode formed of gold and titanium, a silver electrode, a multilayer electrode formed of silver and chromium, a multilayer electrode formed of silver and titanium, a lead electrode, a platinum electrode, a carbon electrode, and the like. It is preferable that a roughening treatment is applied to the surface of first electrode 11 on which cancer cell-containing polymer membrane 5 is formed. By this roughening treatment, the adhesiveness to cancer cell-containing polymer membrane 5 is improved, so that polymer membrane for cancer cell detection 1 can be produced with stability. Furthermore, the roughening treatment can produce an effect of increasing the surface area of the electrode. For example, when a gold electrode is used as first electrode 11, plasma etching is applied to the surface of the gold electrode, on which gold nanoparticles are then fixed, so that a roughening treatment can be implemented. The surface roughness of first electrode 11 is, for example, 0.4 $\mu$m to 50 $\mu$m in terms of center line average roughness.

<Cancer Cell Detection Device>

The cancer cell detection device according to the present invention includes the above-described polymer membrane for cancer cell detection 1. Thus, according to the cancer cell detection device of the present invention, target cancer cell 2 can be immediately and easily detected in an unlabeled manner (that is, directly without using a labeled substance), and also can be detected with excellent specificity.

The cancer cell detection device according to the present invention serves to detect target cancer cells 2 based on the state where target cancer cells 2 are captured in molds 1a, and preferably further includes, in addition to a detection unit including the above-described polymer membrane for cancer cell detection 1, a sensing unit for sensing the state where target cancer cells 2 are captured in molds 1a. The sensing unit for sensing the state where target cancer cells 2 are captured in molds 1a can be configured to sense, for example, a mass change of polymer membrane for cancer cell detection 1 caused by capturing target cancer cells 2, a change in electric conductivity, a change in electric capacity, a change in optical reflectance, a temperature change, or the like. By the method sensing the captured state as described above, target cancer cells 2 can be immediately and easily detected. Specific examples of the method of sensing a mass change may be a method of utilizing a crystal oscillator, and more specifically a method of sensing a change in the resonance frequency of the crystal oscillator.

Target cancer cell 2 can be captured (incorporated) in mold 1a of polymer membrane for cancer cell detection 1 provided in the detection unit, specifically, by bringing the sample solution containing target cancer cells 2 into contact with the surface provided with molds 1a in polymer membrane for cancer cell detection 1. In this case, the driving force causing target cancer cells 2 to move toward the above-mentioned surface may be the electrostatic interaction between negatively-charged target cancer cell 2 and positively-charged polymer membrane for cancer cell detection 1; precipitation of target cancer cells 2 on the above-described surface; diffusion of target cancer cells 2 into the sample solution; and the like. However, in order to allow target cancer cells 2 to be detected more efficiently and more immediately with further excellent detection sensitivity, it is preferable that the cancer cell detection device according to the present invention further includes a guiding unit for guiding target cancer cells 2 contained in the sample solution toward the above-described surface. Such a guiding unit for facilitating movement of target cancer cells 2 to the detection unit may be implemented, for example, by utilizing dielectrophoresis, by utilizing electrophoresis, by generating a flow in a sample solution, and the like.

Dielectrophoresis (DEP) is a phenomenon in which particles (minute substances) within an uneven electric field are moved by the interaction between a specific electric field and the dipole moment induced thereby. Dielectrophoresis can be caused by applying an AC voltage between a pair of electrodes.

Due to the actuation of the guiding unit, target cancer cells 2 contained in the sample solution (in this case, however, the sample solution may not contain target cancer cells 2) are guided toward polymer membrane for cancer cell detection 1 in the detection unit. In this case, not only target cancer cells 2 but also other minute substances like non-target cancer cells are guided toward polymer membrane for cancer cell detection 1. However, target cancer cell 2 having a steric structure complementary to the three-dimensional structure of mold 1a is selectively captured in mold 1a, but other minute substance like a non-target cancer cell not complementary to mold 1a is not captured in mold 1a.

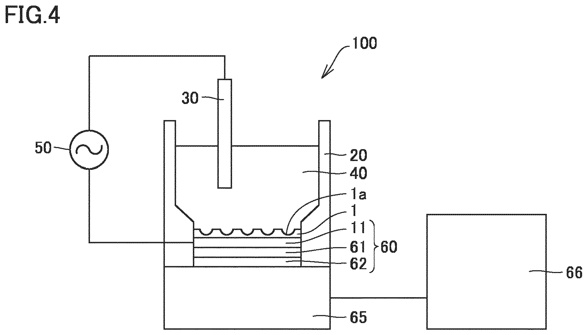

Fig. 4 shows a schematic configuration of a preferable example of the cancer cell detection device according to the present invention. Cancer cell detection device 100 shown in Fig. 4 includes a guiding unit utilizing dielectrophoresis and a sensing unit utilizing a crystal oscillator, in addition to the detection unit including polymer membrane for cancer cell detection 1.

More specifically, cancer cell detection device 100 includes: a cell (reservoir bath) 20 containing sample solution 40; a polymer membrane for cancer cell detection 1 as a detection unit disposed so as to be in contact with sample solution 40 at the bottom of cell 20; a guiding unit utilizing dielectrophoresis and formed of (i) a first electrode 11 stacked on the surface of polymer membrane for cancer cell detection 1 on the side opposite to that provided with mold 1a, (ii) a second electrode (first counter electrode) 30 immersed in sample solution 40, and (iii) an AC power supply 50 connected to first electrode 11 and second electrode 30; and a sensing unit utilizing a crystal oscillator and formed of (i) a crystal oscillator 60 as a stacked body formed by stacking first electrode 11 (which is also used as one of electrodes in the guiding unit), a crystal piece 61 and a third electrode (a second counter electrode) 62, (ii) an oscillation circuit 65, and (iii) a controller 66 including a frequency counter. First electrode 11 can be the first electrode used in the above-described method of producing polymer membrane for cancer cell detection 1 without modification.

Crystal oscillator 60 is an element applied to the Quartz Crystal Microbalance (QCM) method. The QCM method is implemented by utilizing the quantitatively decreasing characteristics of the resonance frequency oscillated in accordance with the mass of the substance adhering to the electrode of the crystal oscillator, to monitor a minute mass change from a picogram level to a nanogram level as a resonance frequency change, thereby measuring the mass change in real time.

The cancer cell detection method using cancer cell detection device 100 is, for example, implemented as follows. First, sample solution 40 is added into cell 20. Then, an AC voltage is applied by AC power supply 50 between one pair of electrodes, that is, between first electrode 11 and second electrode 30, thereby guiding target cancer cells 2 contained in sample solution 40 by dielectrophoresis toward the surface provided with molds 1a in polymer membrane for cancer cell detection 1 serving as a detection unit. In addition, the configuration and the shape of second electrode 30, the voltage to be applied and the frequency value, and the composition of sample solution 40, and the like are appropriately adjusted so as to effectively cause dielectrophoresis.

On the other hand, in response to start of dielectrophoresis, an AC voltage is applied by oscillation circuit 65 between first electrode 11 and third electrode 62, to vibrate crystal piece 61 for monitoring changes in the resonance frequency. When target cancer cell 2 is captured in mold 1a of polymer membrane for cancer cell detection 1, the mass of the substance placed on crystal oscillator 60 increases, thereby decreasing the resonance frequency of crystal oscillator 60 accordingly. In this case, it is assumed that crystal shear stress = 2.95 $\times 10^{11}$ g/cm·sec$^2$, reference frequency = 9.00 MHz, and overtone order = 3. Thus, on the conditions that surface area of crystal oscillator 60 = 0.196 cm$^2$ and density of crystal oscillator 60 = 2.65 g/cm$^3$, the relation between mass change $\Delta m$ (pg) and resonance frequency change $\Delta F$ (Hz) is expressed by the following formula:

$$\Delta m = -357 \Delta F.$$

In response to reception of a signal from oscillation circuit 65, the frequency counter within controller 66 measures a resonance frequency value. Based on the changes in the resonance frequency value, a mass change and consequently the captured state of target cancer cells 2 can be detected.

In addition, a device similar to the device shown in Fig. 4 can be used to allow execution of a roughening treatment of the surface of first electrode 11, formation of cancer cell-containing polymer membrane 5, a peroxidation treatment of cancer cell-containing polymer membrane 5, and the like. For example, cancer cell-containing polymer membrane 5 can be formed by adding, into cell 20, liquid 12 containing target cancer cells 2 and monomers in place of sample solution 40, and connecting a direct-current (DC) power supply in place of AC power supply 50 for causing an electrolytic polymerization reaction. In this case, in addition to first electrode 11, a reference electrode may be separately immersed in liquid 12, thereby allowing potential adjustment. A device including crystal oscillator 60 may be used to form cancer cell-containing polymer membrane 5. Thereby, the thickness of cancer cell-containing polymer membrane 5 to be formed can be monitored based on the changes in the resonance frequency value. Accordingly, the thickness of the resultant polymer membrane for cancer cell detection 1 can be readily controlled.

Similarly, also in the peroxidation treatment of cancer cell-containing polymer membrane 5, in place of sample solution 40, a liquid in a range from a neutral level to an alkaline level (or a product obtained by adjusting liquid 12 obtained after formation of cancer cell-containing polymer membrane 5 so as to fall within a range from a neutral level to an alkaline level) may be added into cell 20, to connect a DC power supply in place of AC power supply 50, thereby applying a DC voltage. Also in this case, in addition to first electrode 11, a reference electrode may be separately immersed in liquid 12, thereby allowing a potential adjustment. The peroxidation treatment can be carried out using a device including crystal oscillator 60, so that the de-doping rate (the removal rate of target cancer cells 2 in cancer cell removal step S102) can be monitored and also can readily be controlled.

According to the cancer cell detection device of the present invention, a target cancer cell can be detected in about several minutes to about several tens of minutes. Thus, a target cancer cell can be much more immediately and easily detected as compared with the CTC test method. As compared with the CTC test method, the cancer cell detection device and the detection method according to the present invention are advantageous also in that a labeled substance is not used, and also, the operator conducting the detection operation is not necessarily a skilled technician.

The polymer membrane of the present invention provided with a mold having a three-dimensional structure complementary to a portion of the steric structure of a target cancer cell can be essentially used for detecting a cancer cell, but can also be used for recovery, removal, concentration, and quantitative determination of cancer cells, identification of cancer cell types, and the like. Furthermore, the polymer membrane of the present invention can be used also for monitoring the transformation from a normal cell to a cancer cell. Furthermore, the polymer membrane of the present invention can be used also for detecting shape abnormality in red blood cells or leukocytes.

Examples

Although the present invention will be hereinafter further specifically described with reference to Examples, the present invention is not limited to these Examples.

In the following Examples,

- Cancer cell-containing polymer membrane 5 and polymer membrane for cancer cell detection 1 were produced using an electrochemical measurement system ("Model 842B" manufactured by ALS) as a potentiostat.
- Ag/AgCl (saturated KCl) was used for a reference electrode, and a Pt rod (having a diameter of 1 mm and a length of 4 cm, and manufactured by Nilaco Corporation) was used for the second electrode (first counter electrode) 30. The electric potential described in the following Examples will be a value relative to the electric potential of this reference electrode.
- Crystal oscillator 60 was manufactured using a stacked body formed of first electrode 11, crystal piece 61 and third electrode (second counter electrode) 62 (electrode area of 0.196 cm$^2$, density = 2.65 g/cm$^3$, fundamental vibration frequency of 9 MHz, crystal shear stress = 2.95 × 10$^{11}$g/cm•sec$^2$, overtone order = 3, AT cut, square shape, and manufactured by SEIKO EG&G). Each of first electrode 11 and third electrode 62 was obtained by stacking a Ti layer (100-nm thickness) and an Au layer (300-nm thickness) in this order from the crystal piece 61 side.
- The resonance frequency change (mass change) during production of cancer cell-containing polymer membrane 5 and polymer membrane for cancer cell detection 1 was measured using a crystal oscillator microbalance ("QCM934" manufactured by SEIKO EG&G).
- An AC voltage was generated using a waveform generator (7075, Hioki, Japan) and an amplifier (HAS 4101, NF, Japan).
- All of the used water was ultrapure water obtained by conducting filtering, pH control, reverse osmosis membrane, ion exchange, and ultraviolet sterilization treatments.

<Example 1>

(1) Roughening Treatment of First Electrode

The surface (Au layer surface) of first electrode 11 of crystal oscillator 60 was subjected to a roughening treatment for the purpose of improving the adhesiveness to a peroxidized polypyrrole film, according to the following procedure.

1. The Au layer surface was subjected to an etching treatment for 30 seconds using plasma etching equipment (SEDE/meiwa fosis).

2. Crystal oscillator 60 was placed on the bottom of cell 20 (a well-type cell, "QA-CL4" manufactured by SEIKO EG&G) in a device similar to that shown in Fig. 4. Then, 500 $\mu$L of solution containing citric acid protected gold nanoparticles (0.0574 wt%) each having a particle size of 30 nm was added to cell 20, and left standing at room temperature for 24 hours, and then, caused the gold nanoparticles to adhere to the surface of first electrode 11.

3. After the surface of first electrode 11 was washed with water, 9 mL of hexadecyltrimethylammonium bromide aqueous solution (0.1M), 250 $\mu$L of gold chloride (III) acid tetrachloride aqueous solution (0.01M), 50 $\mu$L of NaOH aqueous solution (0.1M), and 50 $\mu$L of ascorbic acid aqueous solution (0.1M) were mixed to obtain 500 $\mu$L of solution, which was then added to cell 20 and left standing at 30 °C for 24 hours, so that the gold nanoparticles were fixed.

4. The solution contained in cell 20 was removed and first electrode 11 was washed with water.

(2) Restoration and Culturing of Cancer Cells

As cancer cells, frozen samples of CCRF-CEM (human T lymphocyte leukemia derived cell lines; RBRC-RCB1980) were purchased from RIKEN BRC CELL BANK, and restored and cultured in the following procedure. All of the following operations were performed inside a clean bench.

1. First, 100 mL of fetal bovine serum (FBS) (Gibco) inactivated by incubation for 30 minutes at 56 °C was added to 900 mL of a cell culture solution (RPMI1640, Gibco) and left standing at room temperature for about one day, to prepare a cell culture solution containing serum (RPMI (+)). 2. Then, 1 mL of dissolved product of the above-mentioned frozen samples was dispersed in 9 mL of RPMI (+), which was then subjected to centrifugation (at 1500 rpm for 3 minutes). After that, a supernatant liquid was removed by decantation. Then, 10 mL of RPMI (+) was added to a precipitate (cells), which was then suspended by pipetting. A dish containing the cells was cultured for about one to two days within a $CO_2$ incubator ($CO_2$ concentration 5%) (initial pH7.4 of RPMI (+) as a culture medium, at a temperature of 37 °C).

When an erythrocytometer (manufactured by Sunlead Glass Corporation) was used to measure the number of cells obtained after culturing, the result was $2.0 \times 10^6$ pieces/mL (before culturing: $2.0 \times 10^5$ pieces/mL). By propidium iodide dyeing, it was confirmed through observation using a confocal laser microscope that the survival rate of the cells after culturing was about 95%. The confocal laser microscope observation was performed using FV1200 (OLYMPUS, Japan).

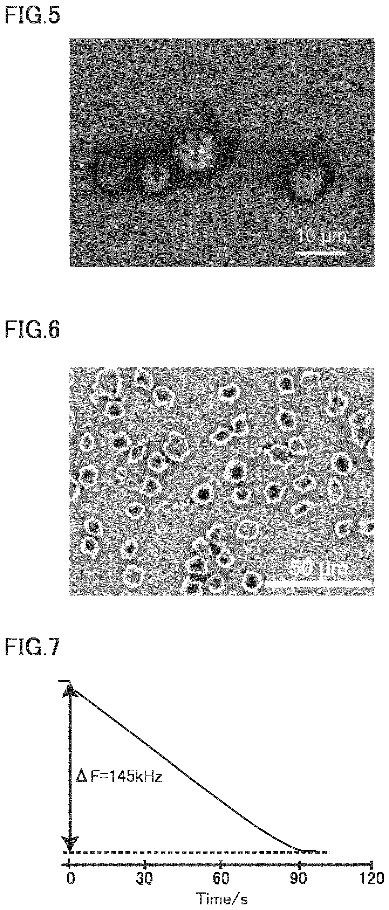

The shape of CCRF-CEM obtained after culturing was observed using a scanning election microscope (SEM, S-4700, Hitachi, Japan). The results are shown in Fig. 5. It was confirmed that CCRF-CEM was a capitulum having a diameter of about 8 $\mu$m.

(3) Preparation of Liquid Containing Target Cancer Cells and Monomers

First, 1.0 mL of cell liquid after culturing obtained in the above (2) was subjected to centrifugation (at 3000 rpm for 1 minute), and then, the obtained supernatant liquid was removed by decantation. The precipitate (cells) obtained after decantation and 0.5 mL of a 0.1M pyrrole aqueous solution containing a phosphate buffer solution (0.2M, pH 2.56) were mixed together to obtain a liquid 12 containing target cancer cells (CCRF-CEM) and pyrrole.

(4) Production of Polymer Membrane for Cancer Cell Detection

According to the following procedure, a peroxidized polypyrrole film as a polymer membrane for cancer cell detection 1 was formed on first electrode 11 of crystal oscillator 60.

1. First, 500 $\mu$L of liquid 12 prepared in the above (3) was added to cell 20 of a device similar to that shown in Fig. 4 in which crystal oscillator 60 subjected to a roughening treatment in the above (1) was disposed on the bottom of cell 20 (but using a DC power supply in placed of AC power supply 50 and including a reference electrode). Then, second electrode 30 and a reference electrode were immersed in liquid 12.

2. First electrode 11 was used as a working electrode to perform constant potential electrolysis (+0.975V), thereby forming, on first electrode 11, a cancer cell-containing polymer membrane 5 (a polypyrrole film) having target cancer cells incorporated therein (polymerization step). During the polymerization step, a crystal oscillator microbalance was used to monitor a resonance frequency change $\Delta$F of crystal oscillator 60, and then, perform constant potential electrolysis until resonance frequency change $\Delta$F was set at -145 kHz.

3. Then, 500 $\mu$L of non-ionic surfactant (Triton-X, 10wt%) was dripped onto the produced cancer cell-containing polymer membrane 5 and left standing at room temperature for about one day, and then, 500 $\mu$L of 5 mg/mL heparin sodium aqueous solution was further dripped thereon and left standing at room temperature for 3 hours to execute a positive charge relaxing treatment for the polymer membrane. Then, the solution within cell 20 was completely removed and the polypyrrole film was washed with phosphate buffered saline (PBS).

4. Then, 0.1 M of NaOH aqueous solution was added into cell 20, in which second electrode 30 and a reference electrode were immersed. First electrode 11 was used as a working electrode to apply +0.975V of a constant potential for 20 minutes, thereby performing a peroxidation treatment to obtain a polymer membrane for cancer cell detection 1 formed of a peroxidized polypyrrole film. During the peroxidation treatment, a crystal oscillator microbalance was used to monitor a resonance frequency change $\Delta F$ of crystal oscillator 60. The thickness of polymer membrane for cancer cell detection 1 measured by a laser microscope was 2 $\mu$m.

(5) Results about Production of Polymer Membrane for Cancer Cell Detection

Fig. 6 shows an electron micrograph of the surface of a polypyrrole film (cancer cell-containing polymer membrane 5) obtained after the polymerization step. The above-described scanning election microscope was used as an electron microscope. It was observed that CCRF-CEM has been incorporated into the surface of the polypyrrole film. When the number of incorporated cells was calculated from the electron micrograph, the result was $1.6 \times 10^6$ pieces/cm$^2$.

Fig. 7 is a graph showing the relation between the resonance frequency of crystal oscillator 60 and the time in the polymerization step. The time at which the constant potential electrolysis was started was set at 0 second. In Fig. 7, the vertical axis shows a resonance frequency. Resonance frequency change $\Delta F$ in the polymerization step was -145 kHz. Thereby, based on the relational expression between the above-mentioned mass change $\Delta m$ and resonance frequency change $\Delta F$, the mass of cancer cell-containing polymer membrane 5 is calculated as 51.8 $\mu$g.

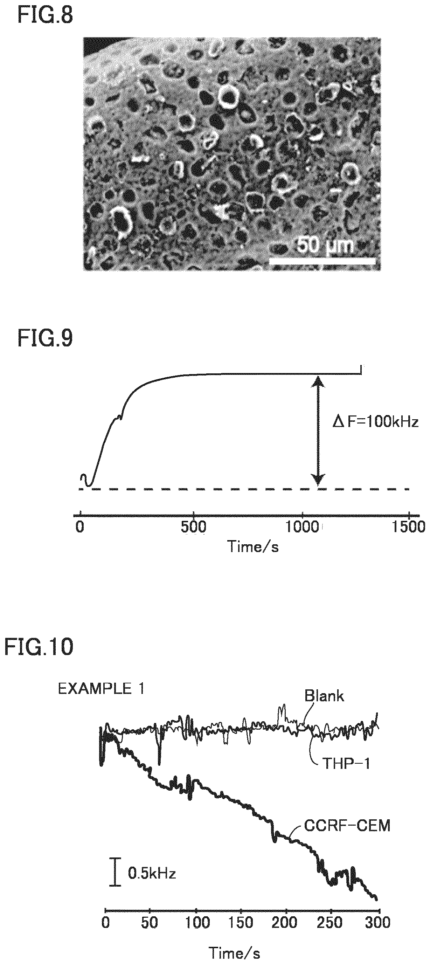

Fig. 8 shows an electron micrograph of the surface of the peroxidized polypyrrole film (a polymer membrane for cancer cell detection 1) obtained after the peroxidation step. The cells incorporated in the mold are less observed, but molds of the cells are formed on the membrane surface. When the number of molds is calculated from the electron micrograph (size: 400 $\mu$m $\times$ 300 $\mu$m), the result is $1.5 \times 10^6$ pieces/cm$^2$, which corresponds to 93% of the number of incorporated cells (de-doping rate = 93%).

Fig. 9 is a graph showing the relation between the resonance frequency of crystal oscillator 60 and the time in the peroxidation step. The time at which the constant potential was applied in the peroxidation step was set at 0 second. In Fig. 9, the vertical axis shows a resonance frequency. Upon progress of peroxidation of the polypyrrole film having CCRF-CEM incorporated therein, the resonance frequency of crystal oscillator 60 was increased as time progressed. Since the resonance frequency is increased by 100 kHz, the degreased amount of the mass of the polymer membrane is calculated as 35.7 $\mu$g based on the relational expression between the above-mentioned mass change $\Delta m$ and resonance frequency change $\Delta F$. It is understood that this is because CCRF-CEM was released from the mold.

(6) Experiment of Detecting Target Cancer Cell

An experiment of detecting CCRF-CEM as a target cancer cell was conducted in the following procedure using a cancer cell detection device having a configuration shown in Fig. 4 and including a peroxidized polypyrrole film (polymer membrane for cancer cell detection 1) obtained in the above (4). First, 500 $\mu$L of cell liquid (cell concentration: $2.0 \times 10^6$ pieces/mL) after culturing obtained in the above (2) was added into cell 20. Then, an AC voltage was applied for 300 seconds between first electrode 11 and second electrode 30, to guide the CCRF-CEM by dielectrophoresis to the surface of polymer membrane for cancer cell detection 1 that is provided with molds. Also, during application of the AC voltage, a crystal oscillator microbalance was used to monitor a resonance frequency change $\Delta F$ of crystal oscillator 60. Resonance frequency change $\Delta F$ was calculated by (i) setting, as a base line, a blank obtained when PBS was added in place of a cell liquid, and (ii) subtracting, from this baseline, a value obtained after 300 seconds since the start of measurement (after application of an AC voltage). As to the AC voltage, a waveform generator (7075, Hioki, Japan) was used to generate an AC voltage (waveform: a sine wave, voltage: 0.2Vpp, frequency: 10 MHz), which was then amplified 10-fold using an amplifier (HAS 4101, NF, Japan) to obtain 2Vpp of an AC voltage, which was then applied.

Furthermore, the detection experiment was conducted in the same manner as described above except for adding a cell liquid after culturing obtained in (2) in Example 2 described later (a cell liquid containing THP-1 that is a non-target cancer cell as a cancer cell) in place of adding a cell liquid after culturing obtained in the above (2).

(7) Results of Experiment of Detecting Target Cancer Cell

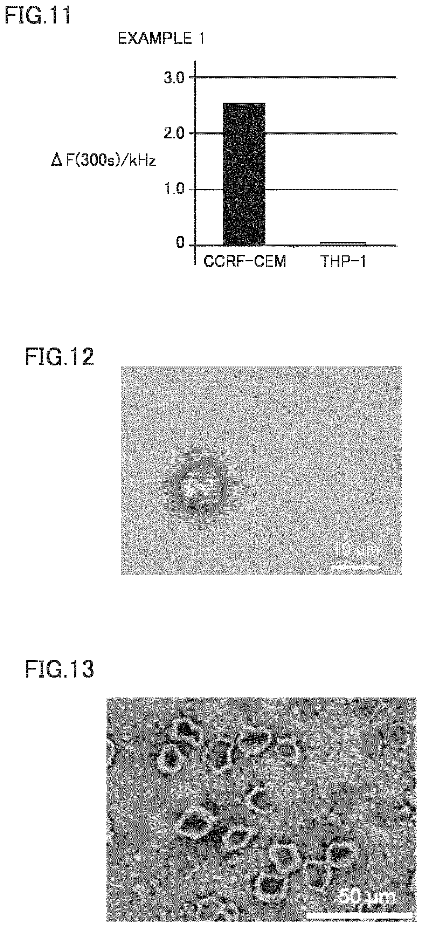

The results of the above-described detection experiment are shown in Figs. 10 and 11. Fig. 10 is a graph showing the relation between the AC-voltage application time and the resonance frequency of the crystal oscillator during detection of each cancer cell (CCRF-CEM or THP-1), and also showing the results of a blank at the time when PBS was added in place of the cell liquid. In Fig. 10, the vertical axis shows a resonance frequency. Fig. 11 is a graph showing a comparison between a resonance frequency change $\Delta F$ for 300 seconds of the AC-voltage application time in the case where a cancer cell is a target cancer cell CCRF-CEM and a resonance frequency change $\Delta F$ for 300 seconds of the AC-voltage application time in the case where a cancer cell is a non-target cancer cell THP-1.

As shown in Figs. 10 to 11, when CCRF-CEM was added to the polymer membrane containing CCRF-CEM as a target cancer cell, the resonance frequency significantly decreased. On the other hand, when THP-1 as a non-target cancer cell was added, the resonance frequency showed little change as in the case of a blank. A decrease in the resonance frequency means an increase in mass of the surface of the crystal oscillator. Also, an increase in mass caused by addition of CCRF-CEM means that CCRF-CEM matching with a mold has been incorporated. On the other hand, when THP-1 not matching with a mold was added, cancer cells have been hardly incorporated into molds. In this way, it can be determined that polymer membrane for cancer cell detection 1 in the present Example allows recognition of the type of the cancer cell with high accuracy (allows specific detection of a target cancer cell).

<Example 2>

(1) Roughening Treatment of First Electrode

The surface of first electrode 11 of crystal oscillator 60 was subjected to a roughening treatment in the same manner as that in Example 1.

(2) Restoration and Culturing of Cancer Cell

As cancer cells, frozen samples of THP-1 (human acute monocytic leukemia; RBRC-RCB1189) were purchased from RIKEN BRC CELL BANK, and then restored and cultured in the procedure similar to that in Example 1.

When an erythrocytometer (manufactured by Sunlead Glass Corporation) was used to measure the number of cells after culturing, the result was $1.0 \times 10^6$ pieces/mL. By propidium iodide dyeing, it was confirmed through observation using a confocal laser microscope that the survival rate of the cells obtained after culturing was about 95%. Also, the shape of THP-1 obtained after culturing was observed using the above-described scanning election microscope. The results thereof are shown in Fig. 12. It was confirmed that THP-1 was a capitulum having a diameter of about 10 $\mu$m.

(3) Preparation of Liquid Containing Target Cancer Cells and Monomers

A liquid 12 containing target cancer cells (THP-1) and pyrrole was obtained in the same manner as that in Example 1 except for using 1.0 mL of cell liquid after culturing obtained in the above (2).

(4) Production of Polymer Membrane for Cancer Cell Detection

In the same manner as that in Example 1 except for using liquid 12 prepared in the above (3), cancer cell-containing polymer membrane 5 (polypyrrole film) having target cancer cells incorporated therein was produced, and then, a polymer membrane for cancer cell detection 1 formed of a peroxidized polypyrrole film was produced. In the present Example, during the polymerization step, a crystal oscillator microbalance was used to monitor a resonance frequency change ΔF of crystal oscillator 60, and then, perform constant potential electrolysis until resonance frequency change ΔF was set at -140 kHz The thickness of polymer membrane for cancer cell detection 1 measured by a laser microscope was 2 $\mu$m.

(5) Results about Production of Polymer Membrane for Cancer Cell Detection

Fig. 13 is an electron micrograph of the surface of a polypyrrole film (cancer cell-containing polymer membrane 5) obtained after the polymerization step. The above-described scanning election microscope was used as an electron microscope. It was observed that THP-1 has been incorporated into the surface of the polypyrrole film. When the number of incorporated cells was calculated from the electron micrograph, the result was $6.7 \times 10^5$ pieces/cm$^2$.

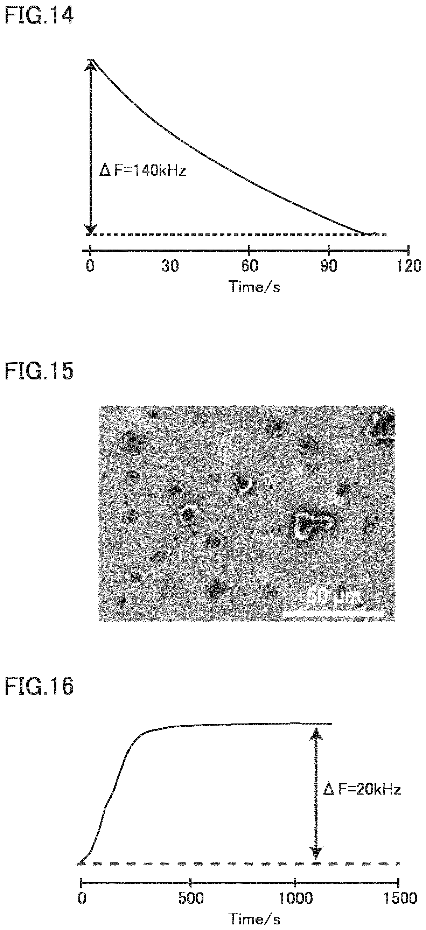

Fig. 14 is a graph showing the relation between the resonance frequency of crystal oscillator 60 and the time in the polymerization step. The time at which the constant potential electrolysis was started was set at 0 second. In Fig. 14, the vertical axis shows a resonance frequency. Resonance frequency change ΔF in the polymerization step was -140 kHz. Thereby, based on the relational expression between the above-mentioned mass change Δm and resonance frequency change ΔF, the mass of cancer cell-containing polymer membrane 5 is calculated as 50.0 $\mu$g.

Fig. 15 is an electron micrograph of the surface of a peroxidized polypyrrole film (polymer membrane for cancer cell detection 1) obtained after the peroxidation step. The cells incorporated in the mold are less observed, but molds of the cells are formed on the membrane surface. When the number of molds is calculated from the electron micrograph (size: 400 $\mu$m $\times$ 300 $\mu$m), the result is $6.0 \times 10^5$ pieces/cm$^2$, which corresponds to 90% of the number of incorporated cells (de-doping rate = 90%).

Fig. 16 is a graph showing the relation between the resonance frequency of crystal oscillator 60 and the time in the peroxidation step. The time at which the constant potential was applied in a peroxidation step was set at 0 second. In Fig. 16, the vertical axis shows a resonance frequency. Upon progress of peroxidation of the polypyrrole film having THP-1 incorporated therein, the resonance frequency of crystal oscillator 60 was increased as time progressed. Since the resonance frequency is increased by 20 kHz, the degreased amount of the mass of the polymer membrane is calculated as 7.14 $\mu$g based on the relational expression between the above-mentioned mass change Δm and resonance frequency change ΔF. It is understood that this is because THP-1 was released from the mold.

(6) Experiment of Detecting Target Cancer Cell

In the same manner as that in Example 1 except for using THP-1 as a target cancer cell, an experiment of detecting a target cancer cell was conducted using a cancer cell detection device having a configuration shown in Fig. 4 and including a peroxidized polypyrrole film (polymer membrane for cancer cell detection 1) obtained in the above (4). As a cell liquid added into cell 20, the cell liquid after culturing obtained in the above (2) (cell concentration: $1.0 \times 10^6$ pieces/mL) was used.

The detection experiment was conducted in the same manner as described above except that a cell liquid after culturing obtained in (2) in Example 1 (a cell liquid containing CCRF-CEM corresponding to a non-target cancer cell as a cancer cell) was added in place of adding the cell liquid after culturing obtained in the above (2).

(7) Results of Experiment of Detecting Target Cancer Cell

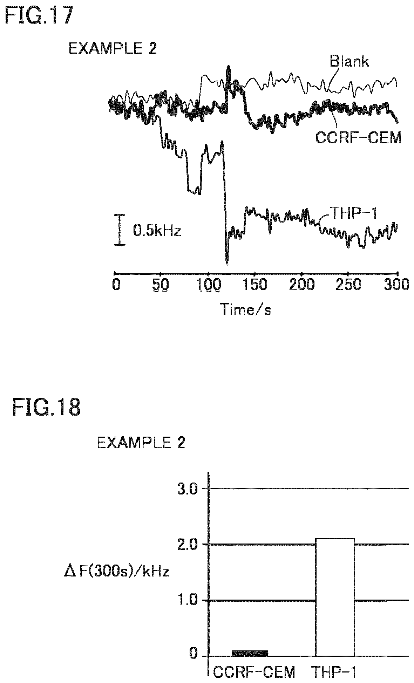

The results of the above-described detection experiment are shown in Figs. 17 and 18. Fig. 17 is a graph showing the relation between the AC-voltage application time and the resonance frequency of a crystal oscillator during detection of each cancer cell (THP-1 or CCRF-CEM) and also showing the results of a blank at the time when PBS was added in place of a cell liquid. In Fig. 17, the vertical axis shows a resonance frequency. Fig. 18 is a graph showing a comparison between a resonance frequency change ΔF for 300 seconds of the AC-voltage application time in the case where the cancer cell is a target cancer cell THP-1 and a resonance frequency change ΔF for 300 seconds of the AC-voltage application time in the case where the cancer cell is a non-target cancer cell CCRF-CEM.

As shown in Figs. 17 and 18, in the case where THP-1 was added to the polymer membrane containing THP-1 as a target cancer cell, the resonance frequency significantly decreased. On the other hand, in the case where CCRF-CEM as a non-target cancer cell was added, the resonance frequency showed little change as in the case of a blank. A decrease in the resonance frequency means an increase in mass of the surface of the crystal oscillator. Also, an increase in mass caused by addition of THP-1 means that THP-1 matching with a mold has been incorporated. On the other hand, in the case where CCRF-CEM not matching with a mold was added, cancer cells have been hardly incorporated into molds. In this way, it can be determined that polymer membrane for cancer cell detection 1 in the present Example allows recognition of the type of the cancer cell with high accuracy (allows specific detection of target cancer cells).

REFERENCE SIGNS LIST

1 polymer membrane for cancer cell detection, 1a mold, 2 target cancer cell, 5 cancer cell-containing polymer membrane, 11 first electrode, 12 liquid containing target cancer cells and monomers, 20 cell, 30 second electrode (first counter electrode), 40 sample solution, 50 AC power supply, 60 crystal oscillator, 61 crystal piece, 62 third electrode (second counter electrode), 65 oscillation circuit, 66 controller, 100 cancer cell detection device, A thickness of polymer membrane for cancer cell detection.

Claims

1. A polymer membrane for cancer cell detection, the polymer membrane having a surface provided with a mold having a three-dimensional structure complementary to a portion of a steric structure of a cancer cell to be detected.

2. The polymer membrane according to claim 1, wherein a thickness of the polymer membrane is 5 μm or less.

3. The polymer membrane according to claim 1, wherein the polymer membrane is obtained by a producing method including:

polymerizing monomers in presence of cancer cells to be detected, to form a cancer cell-containing polymer membrane having the cancer cells incorporated therein; and
removing at least part of the cancer cells incorporated in the cancer cell-containing polymer membrane.

4. The polymer membrane according to claim 3, wherein the removing includes, in a following order:

decreasing a volume of the cancer cells incorporated in the cancer cell-containing polymer membrane or at least partially neutralizing an electrostatic interaction between the cancer cells and a polymer membrane forming the cancer cell-containing polymer membrane; and
peroxidizing the cancer cell-containing polymer membrane to release at least part of the cancer cells incorporated in the cancer cell-containing polymer membrane.

5. The polymer membrane according to claim 4, wherein
the decreasing a volume of the cancer cells incorporated in the cancer cell-containing polymer membrane or at least partially neutralizing an electrostatic interaction between the cancer cells and a polymer membrane forming the cancer cell-containing polymer membrane includes
treating the cancer cell-containing polymer membrane with heparin sodium.

6. The polymer membrane according to claim 3, wherein the monomers are selected from the group consisting of pyrrole, aniline, thiophene, and a derivative thereof.

7. A cancer cell detection device comprising:

a detection unit including the polymer membrane according to claim 1;
a guiding unit for guiding a cancer cell to be detected, which is contained in a sample solution, toward the surface of the polymer membrane; and
a sensing unit for sensing a state where the cancer cell to be detected is captured in the mold.

8. The cancer cell detection device according to claim 7, wherein the guiding unit is configured to apply an alternating-current (AC) voltage between a pair of electrodes to guide the cancer cell to be detected toward the surface of the polymer membrane by dielectrophoresis.

9. The cancer cell detection device according to claim 7, wherein
the sensing unit includes a crystal oscillator, and
the sensing unit is configured to measure a mass change of the polymer membrane based on a change of a resonance frequency of the crystal oscillator, to sense the state where the cancer cell to be detected is captured.

10. A producing method of a polymer membrane for cancer cell detection, the polymer membrane having a surface provided with a mold having a three-dimensional structure complementary to a portion of a steric structure of a cancer cell to be detected, the producing method comprising:

polymerizing monomers in presence of cancer cells to be detected, to form a cancer cell-containing polymer membrane having the cancer cells incorporated therein; and
removing at least part of the cancer cells incorporated in the cancer cell-containing polymer membrane.

11. The producing method according to claim 10, wherein, the removing comprises, in a following order:

decreasing a volume of the cancer cells incorporated in the cancer cell-containing polymer membrane or at least partially neutralizing an electrostatic interaction between the cancer cells and a polymer membrane forming the cancer cell-containing polymer membrane; and

peroxidizing the cancer cell-containing polymer membrane to release at least part of the cancer cells incorporated in the cancer cell-containing polymer membrane.

12. The producing method according to claim 11, wherein the decreasing a volume of the cancer cells incorporated in the cancer cell-containing polymer membrane or at least partially neutralizing an electrostatic interaction between the cancer cells and a polymer membrane forming the cancer cell-containing polymer membrane includes
treating the cancer cell-containing polymer membrane with heparin sodium.

FIG.1

1a          1a          1a

A

1

FIG.2

2

1

FIG.3

(a)

POLYMERIZATION
STEP(S101)

(b)

CANCER CELL REMOVAL
STEP(S102)

(c)

FIG.4

FIG.5

FIG.6

FIG.7

FIG.8

FIG.9

$\Delta F=100kHz$

Time/s

FIG.10

EXAMPLE 1

Blank

THP-1

CCRF-CEM

0.5kHz

Time/s

FIG.11

EXAMPLE 1

FIG.12

FIG.13

FIG.14

FIG.15

FIG.16

FIG.17

EXAMPLE 2

Time/s

FIG.18

EXAMPLE 2

## INTERNATIONAL SEARCH REPORT

| International application No. |
| --- |
| PCT/JP2015/062955 |

**A. CLASSIFICATION OF SUBJECT MATTER**
G01N33/566(2006.01)i, C12M1/34(2006.01)i, G01N33/543(2006.01)i,
G01N33/574(2006.01)i, C12Q1/04(2006.01)n

According to International Patent Classification (IPC) or to both national classification and IPC

**B. FIELDS SEARCHED**

Minimum documentation searched (classification system followed by classification symbols)
G01N33/48-33/98, C12M1/34, C12Q1/04, G01N27/26-27/49

Documentation searched other than minimum documentation to the extent that such documents are included in the fields searched
Jitsuyo Shinan Koho          1922-1996    Jitsuyo Shinan Toroku Koho    1996-2015
Kokai Jitsuyo Shinan Koho    1971-2015    Toroku Jitsuyo Shinan Koho    1994-2015

Electronic data base consulted during the international search (name of data base and, where practicable, search terms used)
JSTPlus/JMEDPlus/JST7580(JDreamIII), PubMed

**C. DOCUMENTS CONSIDERED TO BE RELEVANT**

| Category* | Citation of document, with indication, where appropriate, of the relevant passages | Relevant to claim No. |
| --- | --- | --- |
| Y | WO 2012/121229 A1 (Osaka Prefecture University), 13 September 2012 (13.09.2012), paragraphs [0003], [0006], [0039] to [0060]; fig. 1 to 3, 6 to 23 & EP 2684946 A1          & US 2013337498 A1 & CN 103459583 A | 1-4,6-11 |
| Y | F. L. Dickert and O. Hayden, Bioimprinting of Polymers and Sol-Gel Phases. Selective Detection of Yeasts with Imprinted Polymers, Analytical Chemistry, 2002.07.02, Vol.74, No.6, p.1302-1306 | 1-4,6-11 |

[X]  Further documents are listed in the continuation of Box C.          [ ]  See patent family annex.

| * | Special categories of cited documents: |
| --- | --- |
| "A" | document defining the general state of the art which is not considered    to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 28 July 2015 (28.07.15) | 04 August 2015 (04.08.15) |

| Name and mailing address of the ISA/ Japan Patent Office 3-4-3, Kasumigaseki, Chiyoda-ku, Tokyo 100-8915, Japan | Authorized officer |
| --- | --- |
| | Telephone No. |

Form PCT/ISA/210 (second sheet) (July 2009)

**INTERNATIONAL SEARCH REPORT**

International application No.

PCT/JP2015/062955

C (Continuation).   DOCUMENTS CONSIDERED TO BE RELEVANT

| Category* | Citation of document, with indication, where appropriate, of the relevant passages | Relevant to claim No. |
|---|---|---|
| A | US 2004/0126814 A1  (Waheguru Pal Singh et al.), 01 July 2004 (01.07.2004), paragraphs [0010], [0011], [0017], [0032], [0046]; example 6; fig. 1, 3, 11 (Family: none) | 1-12 |
| A | Tally Cohen et al, Whole Cell Imprinting in Sol-Gel Thin Films for Bacterial Recognition in Liquids: Macromolecular Fingerprinting, International Journal of Molecular Sciences, 2010.03.24, Vol. 11, NO.5, p.1236-1252 | 1-12 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012121229 A